# EUROPEAN PATENT APPLICATION

(11) **EP 1 441 523 A1**
(43) Date of publication of application: **28.07.2004**
(21) Application number: 04250297.1
(22) Date of filing: 21.01.2004
(51) Int. Cl.: H04N 7/14, G06F 13/40, H04M 1/725, H04N 1/00, H04N 1/21

(54) **Mobile terminal having image processing function and method therefor**

(30) Priority: 21.01.2003 KR 2003003895
(71) Applicant: Curitel Communications, Inc., Ichon-shi, Kyoungki-do 467-860 (KR)
(72) Inventor: Min, Dong-Uk, Ichon-shi Kyoungki-do 467-860 (KR); Lee, In-Sub, Ichon-shi Kyoungki-do 467-860 (KR)
(74) Representative: Mounteney, Simon James

(57) **Abstract**

A mobile terminal having an image processing function that can perform image processing procedure independently from call processing and a method therefor. The mobile terminal includes: an image sensor for obtaining an image; an image digital processing (DSP) unit for formatting the image into specific image format data; an interface unit for transmitting the specific image format data to an external unit; and a control unit for generating a control signal allowing the specific image format data to be transmitted to the external unit.

## Description

### Field of the Invention

The present invention relates to a mobile terminal having a function of a personal computer (PC) camera and a method therefor; and, more particularly, to a mobile terminal having a function of PC camera which can perform image data processing as well as call processing, the basic function of a mobile terminal, independently and further implement multimedia services.

### Description of Related Art

Mobile terminals, e.g., wireless phones have come into wide use. A major role of the wireless phone is a call processing, but the wireless phone is recently demanded to provide various multimedia functions. Recently, video chatting via computer is all the rage among especially young people. Nevertheless, conventional wireless phones have no PC camera function, even though the conventional wireless phone has a camera. The video chatting via computer requires an expensive camera such as a personal computer (PC) camera additionally.

### Summary of the Invention

It is, therefore, an object of the present invention to provide a mobile terminal having an image processing function that can perform an image processing.

In accordance with an aspect of the present invention, there is provided a mobile terminal having an image processing function, including: an image sensor for obtaining an image; an image digital processing (DSP) unit for formatting the image into specific image format data; an interface unit for transmitting the specific image format data to an external unit; and a control unit for generating a control signal allowing the specific image format data to be transmitted to the external unit.

In accordance with another aspect of the present invention, there is provided a method for performing an image processing function in a mobile terminal, including the steps of: a) generating a transmission control signal according to a kind of transmission data to be transmitted; b) if the transmission data is data stored in mobile terminal, transmitting the data through a switching unit to an external unit based on a PC link signal; and c) if the transmission data is image data captured in an image sensor, converting the image data to YUV data, encoding the YUV data to generate encoded data to the external machine through a switching unit based on a PC camera signal.

In accordance with further another aspect of the present invention, there is provided a method of performing an image processing function in a mobile terminal, including the steps of: capturing an image; formatting the image into a specific image format data; and transmitting the specific image format data to an external unit.

### Brief Description of the Drawings

The above and other objects and features of the present invention will become apparent from the following description of the preferred embodiments given in conjunction with the accompanying drawings, in which:
Fig. 1 is a block diagram showing a mobile terminal having an image processing function in accordance with an embodiment of the present invention;
Fig. 2 is a block diagram illustrating an image digital signal processor (DSP) installed in the mobile terminal having an image processing function in accordance with the first embodiment of the present invention;
Fig. 3 is a block diagram showing a device for controlling the clocks of the image DSP installed in the mobile terminal having an image processing function in accordance with the embodiment of the first present invention;
Fig. 4 is a flow chart illustrating an image processing method in a mobile terminal in accordance with the present invention; and
Fig. 5 is a block diagram illustrating a mobile terminal having an image processing function in accordance with another embodiment of the present invention.

### Detailed Description of the Invention

Other objects and aspects of the invention will become apparent from the following description of the embodiments with reference to the accompanying drawings, which is set forth hereinafter.

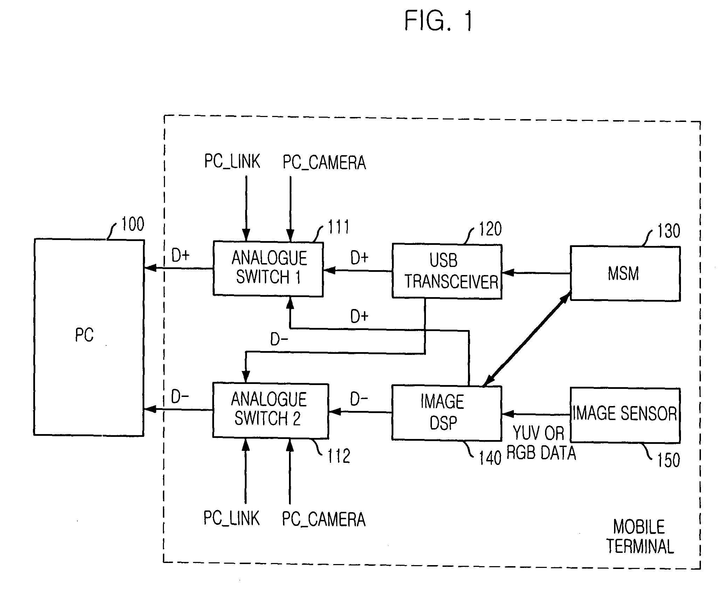

Fig. 1 shows a block diagram showing a mobile terminal having an image processing function in accordance with a first embodiment of the present invention. The mobile terminal having an image processing function of the present invention includes a plurality of analogue switches 111 and 112, a universal serial bus (USB) transceiver 120, a controller such as a mobile station modem (MSM) 130, an image digital signal processor (DSP) 140, and an image sensor 150.

As shown, a personal computer (PC) 100 receives data from the mobile terminal through USB lines (D-, D+) coupled thereto. The embodiment of the present invention presents the PC 100 as an example of an external machine. However, other machines such as a printer, an extension USB memory, a USB keyboard, an audio component and the like, can be used instead of the PC as long as they can function as a USB master. Also, the external machine can be connected to the mobile terminal through other connection lines, for example, RS232C data lines as well as the USB lines.

The analogue switches 111 and 112 are connected to the connection lines coupled with the external machine, respectively, and perform multi-switching operation based on a PC link signal PC_LINK and a PC camera signal PC_CAMERA. When the PC link signal PC_LINK is activated, the analogue switches 111 and 112 provide data paths to the USB transceiver 120. If the PC camera signal PC_CAMERA is activated, the analogue switches 111 and 112 provide data paths to the image DSP 140. The analogue switches 111 and 112 are preferably Single Pole Double Throw (SPDT) switches that maintain an open (off) state when both of the PC link signal PC_LINK and PC camera signal PC_CAMERA are inactivated.

The USB transceiver 120 transmits the data from the MSM 130 to the analogue switches 111 and 112 based on the USB standard.

The MSM 130 converts the data of the mobile terminal into data suitable to the USB interface. The MSM 130 also activates the PC link signal PC_LINK and the PC camera signal PC_CAMERA.

The image DSP 140 processes image data from the image sensor 150 and audio data from the outside of the mobile terminal, and generates preview image data and encoded data. Then, the image DSP 140 outputs the encoded data to the analogue switches 111 and 112 or MSM 130, and outputs the preview image data to be displayed on a liquid crystal display (LCD) device (not shown).

The image sensor 150 captures images of objects from the outside and outputs the image data to the image DSP 140.

In this embodiment of the present invention described above, only for easy description, it is described that the data is transmitted from the mobile terminal to the external machine 100. However, it is well known to ordinary one skilled in the art that the data can be transmitted from the external machine 100 to the mobile terminal. The mobile terminal having an image processing function provides an independent path between the external machine 100 as a master and the mobile terminal as a slave by using the SPDT switches which control the D+/D- path of the respective USB lines in response to the control of the PC link signal PC_LINK or the PC camera signal PC_CAMERA.

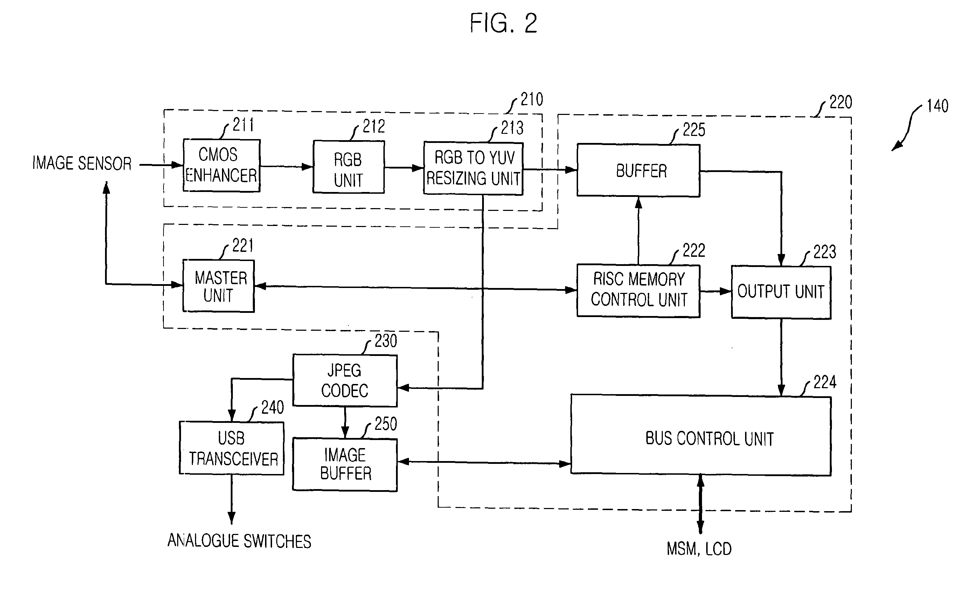

Fig. 2 is a block diagram illustrating an image DSP installed in the mobile terminal having an image processing function in accordance with the embodiment of the present invention.

The image DSP includes a YUV data processing portion 210, an image parallel processing portion 220, a Joint Photographic Coding Experts Group (JPEG) codec 230, a USB transceiver 240 and an image buffer 250.

The YUV data processing portion 210 outputs YUV data to Joint Photographic Coding Experts Group (JPEG) codec 230 and the image parallel processing portion 220. YUV means a color space derived from the phase alternating by line color (PAL) television color standard. Y denotes the luminance channel, U the blue channel, and V the red channel.

The YUV data processing portion 210 includes a complementary metal oxide semiconductor (CMOS) enhancer 211, a red/green/blue (RGB) unit 212 and a RGB to YUV resizing unit 213.

The CMOS enhancer 211 installed in the YUV data processing portion 210 controls the luminance and brightness of the image data having a RGB format transmitted from the image sensor 150 and outputs the resultant data to the RGB unit 212.

The RGB unit 212 of the YUV data processing portion 210 converts the RGB image data from the CMOS enhancer 211 into, for example, 8×8 RGB block data, and outputs the 8×8 RGB block data.

The RGB to YUV resizing unit 213 of the YUV data processing portion 210 receives the block data of the RGB format from the image sensor 150 and converts them into image data having YUV format (hereinafter, which is referred to as "YUV data").

The image parallel processing portion 220 generates output image data based on the YUV data received from the YUV data processing portion 210 which are outputted to the MSM and an LCD device (not shown). To perform the above-described operation, the image parallel processing portion 220 includes a buffer 225, an output unit 223, a bus control unit 224, a reduced instruction set computer (RISC) memory control unit 222 and a master unit 221.

The master unit 221 installed in the image parallel processing portion 220 generates preview image data based on data received from the image sensor 150.

The RISC memory control unit 222 of the image parallel processing portion 220 transmits the preview image data received from the master unit 221 to the output unit 223. However, if the size of the preview image is large, the preview image data is transmitted to the output unit 223 through the buffer 225.

The output unit 223 of the image parallel processing portion 220 outputs the preview image data received from the buffer 225 and the RISC memory control unit 222 to the bus control unit 224.

The bus control unit 224 of the image parallel processing portion 220 outputs the preview image data from the output unit 223 to the LCD device, and provides an image data having a specific format (here, JPEG format) encoded by the JPEG codec 230 through the image buffer 250 to the MSM 130. The MSM 130 stores the image data having the JPEG format in a predetermined space of a memory.

The buffer 225 of the image parallel processing portion 220 stores the data from the RGB to YUV resizing unit 213 and the RISC memory control unit 222, temporarily.

The JPEG codec 230 receives the YUV data from the YUV data processing portion 210, and encodes the YUV data into image data having a JPEG format (which is referred to as "JPEG image data") based on the JPEG method and transmits the JPEG image data to the external machine 100 through the analogue switches 111 and 112. Here, though the JPEG is a compression technique for coding still images, the JPEG codec 230 encodes a plurality of the still images within a predetermined time (e.g., 33 frames per a second), which are transmitted to the external machine 100 in real time and thus are seen as a moving picture to a user. This is well known to the ordinary one skilled in the art.

The USB transceiver 240 transmits the JPEG image data to the external machine 100 based on the USB standard. The USB transceiver 240 performs the same function as the USB transceiver 120. Thus, the USB transceivers 120 and 240 can be can be formed one USB transceiver so that data of both the MSM 130 and the image DSP 140 can be transmitted to a single USB transceiver.

Hereafter, the operation of the image DSP 140 is described in accordance with the present invention. To execute the preview function, the master unit 221 receives image data captured in the image sensor 150 and generates preview image data. Then the RISC memory control unit 222 receives the preview image data from the master unit 221 and transmits the preview image data to the output unit 223. If the size of the preview image is large, the preview image is transmitted to the output unit 223 through the buffer 225. For example, if the user wants to display the preview image having a high resolution, the preview image is encoded into a high resolution image and then transmitted to the output unit 223. Then, the bus control unit 224 receives the data from the output unit 223 and outputs the preview image data to the LCD device.

Meanwhile, to describe the data output process of the external machine 100, first, the CMOS enhancer 211 controls the luminance and brightness of the image data transmitted from the image sensor 150 and outputs the resultant data. Then, the RGB unit 212 converts the data from the CMOS enhancer 211 into 8×8 block data.

The RGB to YUV resizing unit 213 of the YUV data processing portion 210 receives the data of the RGB format from the image sensor 150 and converts them into YUV data. The YUV data is encoded by the JPEG codec 230. In this embodiment, the JPEG is described as a coding(compression)/decoding(decompression) method of the YUV data, it is well known to one skilled in the art that other coding/decoding method, e.g., MPEG-4, HL-26L can be used. The encoded YUV data is transmitted to the external machine through the USB transceiver 240 and the analogue switches 111 and 112, and also transmitted to the MSM through the image buffer 250.

Here, by a selection of a user, the YUV data encoded by the JPEG codec 230 is transmitted to the MSM through the image buffer 250 and stored in a predetermined space of a memory (not shown).

The image DSP 140 may include and control a digital camera having a 330,000-pixel CMOS lens that can process 33 frames per second within a mobile terminal. The image DSP 140 can provide the preview and snap shot function through an LCD device (not shown) and display acquired images on the screen of the external machine 100 through USB data communication.

The CMOS enhancer 211 adjusts gradation of the image data, YUV data (data before compression), inputted from the image sensor 150 based on a gamma control method, controls the brightness of the image data, and transmits the resultant data to the JPEG codec 230. The adjusted image data are compressed by the JPEG codec 230 and transmitted to the external machine 100 through the USB lines. Subsequently, in the external machine 100, the compressed image data are decompressed by the decompression engine installed in the external machine 100, whereby the image captured by the mobile terminal is displayed on a screen of the external machine 100.

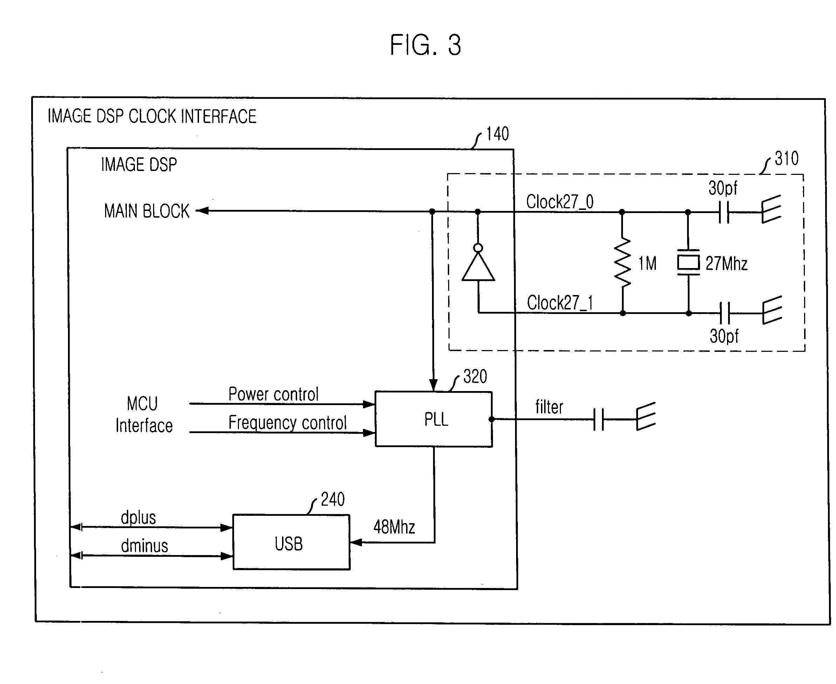

Fig. 3 is a block diagram showing a device for controlling clocks of the image DSP 140 installed in the mobile terminal having an image processing function in accordance with the embodiment of the present invention.

A basic clock generation unit 310 generates a basic clock (for example, 27MHz) for the image DSP 140, and outputs the basic clock to a phase-locked loop (PLL) 320. Here, the basic clock is provided as a clock signal for elements installed in the image DSP 140 except the USB transceiver 240.

The PLL 320 receives the basic clock from the basic clock generation unit 310 and generates an USB clock (48MHz) for the USB transceiver 240 based on the basic clock. The function of generating clocks is installed within the image DSP 140, and thus the hardware space and the cost can be saved.

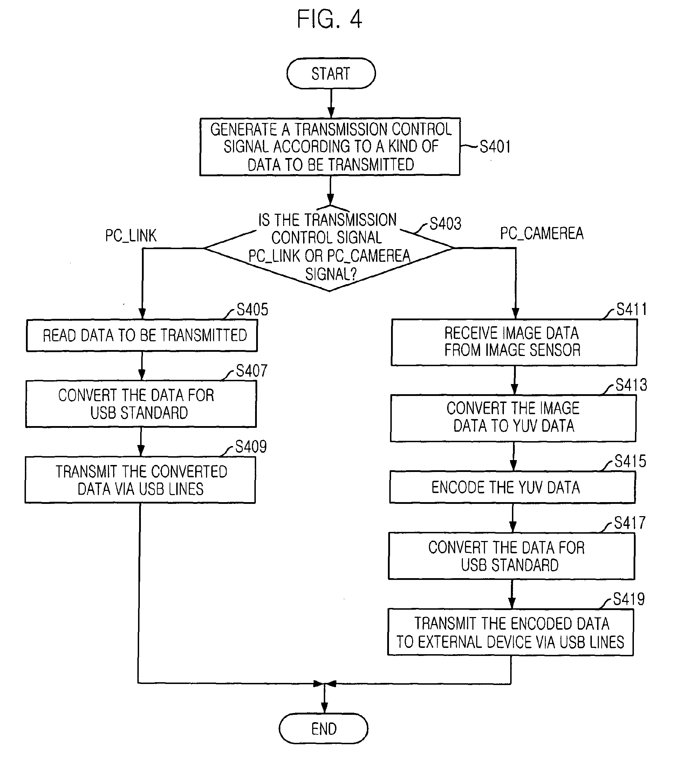

Fig. 4 is a flow chart illustrating an image processing method in a mobile terminal in accordance with the present invention.

At step S401, the MSM 130 generates a transmission control signal according to a kind of transmission data to be transmitted. In other words, if the transmission data is the data stored in the mobile terminal, the MSM 130 generates a PC Link signal PC_LINK, and if the transmission data is the image data captured in the image sensor 150, the MSM 130 generates a PC camera signal PC_CAMERA. At step S403, it is determined whether the transmission control signal is the PC link signal PC_LINK or the PC camera signal PC_CAMERA.

If the transmission data is the data stored in the mobile terminal and the MSM 130 generates the PC link signal PC_LINK, at step S405, the MSM 130 reads the data to be transmitted, and at step S407, the USB transceiver 120 transmits the data to be transmitted based on the USB standard. At step S409, the analogue switches 111 and 112 transmits the converted data to the external machine 100 through the USB lines based on the PC link signal PC_LINK.

If the transmission data is the image data captured in the image sensor 150, the MSM 130 generates a PC camera signal PC_CAMERA, at step S411, the image DSP 140 receives the image data captured in the image sensor 150, and at step S413, converts the image data to YUV data. The YUV data is encoded based on the JPEC coding method at step S415. At step S417, the USB transceiver 240 converts the encoded data for the USB standard, and at step S419 the analogue switches 111 and 112 transmits the converted data to the external machine 100 through the USB lines based on the PC camera signal PC_CAMERA.

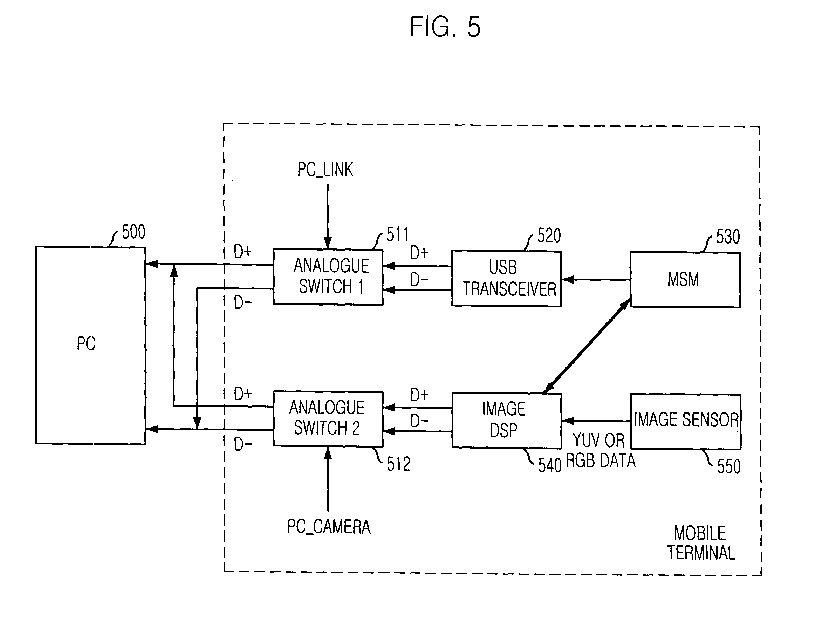

Fig. 5 is a block diagram illustrating a mobile terminal having an image processing function in accordance with a second embodiment of the present invention. The mobile terminal having an image processing function includes a first analogue switch 511, a second analogue switch 512, a USB transceiver 520, a controller such as an MSM 530, an image DSP 640 and an image sensor 550.

As shown, the PC 500 receives data from the mobile terminal through USB lines (D-, D+). The second embodiment of the present invention presents the PC 500 as an example of external machine. However, other machines such as a printer, an extension USB memory, a USB keyboard, an audio component and the like, can be used instead of the PC as long as they can function as a USB master. Also, the external machine can be connected to the mobile terminal through other connection lines, for example, RS232C data lines as well as the USB lines.

The first analogue switch 511 is connected to the external machine 500 through a communication line and performs multi-switching operation based on a PC link signal PC_LINK. If the PC link signal PC_LINK is activated, the first analogue switch 511 provides a data path for the USB transceiver 520. The first analogue switch 511 is preferably a dual SPDT switch, which maintains the open state when the PC link signal PC_LINK is inactivated.

The second analogue switch 512 is connected to the external machine 500 through a communication line and performs multi-switching operation based on a PC camera signal PC_CAMERA. If the PC camera signal PC_CAMERA is activated, the second analogue switch 612 provides a data path for the image DSP 540. The second analogue switch 512 is a dual SPDT switch, which maintains the open state when the PC camera signal PC_CAMERA is inactivated.

The USB transceiver 520 converts the data from the MSM 530 suitable for the USB standard and transmits the converted data to the external machine 500 through the first analogue switch 511 when the PC link signal PC_LINK is activated.

The MSM 530 converts the data of the mobile terminal into data suitable to the USB transceiver 520 interface. The MSM 530 also activates the PC link signal PC_LINK and the PC camera signal PC_CAMERA.

The image DSP 540 receives and processes image data from the image sensor 550 and audio data from the outside of the mobile terminal. Then, the image DSP 540 outputs the processed data to the second analogue switch 512. The image DSP 540 can also display the image data on an LCD device (not shown) and outputs the audio data through an output unit (not shown).

The image sensor 550 acquires images of objects from the outside and outputs them as image data.

In the second embodiment of the present invention described above, the mobile terminal having an image processing function provides an independent path between the external machine 500, a master, and the mobile terminal, a slave, by using SPDT switches to control the D+/D- path of the respective USB lines based on the control of the PC link signal PC_LINK or the PC camera signal PC_CAMERA. The internal circuit configurations of the components described above are the same as those of the first embodiment shown in Fig. 1. Therefore, further description on the second embodiment will be omitted, for the sake of convenience.

The technology of the present invention has an advantage that image processing can be performed independently without carrying a load on the call process procedure by setting up a device for processing images within the mobile terminal and adopting a switching connection method.

Further, in addition to a function that stores an image captured by a camera, the mobile terminal of the present invention provides a PC camera function that directly displays an image captured by a camera on a personal computer. Therefore, a user can enjoy a video chatting without using an additional expensive PC camera.

While the present invention has been described with respect to certain preferred embodiments, it will be apparent to those skilled in the art that various changes and modifications may be made without departing from the scope of the invention as defined in the following claims.

## Claims

1. A mobile terminal having an image processing function, comprising:
an image sensor for obtaining an image;
an image digital processing (DSP) unit for formatting the image into specific image format data;
an interface unit for transmitting the specific image format data to an external unit; and
a control unit for generating a control signal allowing the specific image format data to be transmitted to the external unit.

2. The mobile terminal as recited in claim 1, further comprising: a memory unit for storing the specific image format data.

3. The mobile terminal as recited in claim 2, wherein the control signal includes a first control signal which controls the data stored in the memory unit to be transmitted, and a second control signal which controls the specific image format data to be transmitted.

4. The mobile terminal as recited in claim 3, further comprising:
a switching unit having a plurality of switches, for allowing the specific image format data and the data to be transmitted to the external unit based on the first control signal and the second control signal.

5. The mobile terminal as recited in claim 4, wherein the first control signal is a personal computer (PC) link signal, and the second control signal is a PC camera signal.

6. The mobile terminal as recited in claim 3, further comprising:
a first switch for allowing the data to be transmitted to the external unit based on the first control signal; and
a second switch for allowing the specific image format data to be transmitted to the external unit based on the second control signal.

7. The mobile terminal as recited in claim 6, wherein the first control signal is a personal computer (PC) link signal, and the second control signal is a PC camera signal.

8. The mobile terminal as recited in claim 1, wherein the image DSP unit includes:
a YUV data processing portion for generating YUV data based on the image received from the image sensor ;
an image parallel processing portion for receiving the YUV data and generating preview image data based on the YUV data; and
an encoding unit for encoding the YUV data, generating encoded data, and transmitting the encoded data through the switches based on the PC camera signal.

9. The mobile terminal as recited in claim 8, wherein the encoding unit includes a Joint Photographic Coding Experts Group (JPEG) codec.

10. The mobile terminal as recited in claim 8, wherein the encoding unit includes a Moving Picture Experts Group (MPEG) codec.

11. The mobile terminal having an image processing function as recited in claim 1, wherein the image DSP unit includes:
a basic clock generation unit for generating a basic clock for the image DSP unit; and
a phase locked loop (PLL) for generating a USB clock for the interface unit.

12. A method for performing an image processing function in a mobile terminal, comprising the steps of:
a) generating a transmission control signal according to a kind of transmission data to be transmitted;
b) if the transmission data is data stored in mobile terminal, transmitting the data through a switching unit to an external unit based on a PC link signal; and
c) if the transmission data is image data captured in an image sensor, converting the image data to YUV data, encoding the YUV data to generate encoded data to the external unit through the switching unit based on a PC camera signal.

13. The method as recited in claim 12, further comprising the step of d) if a preview function is selected, displaying the YUV data on a display unit.

14. A method of performing an image processing function in a mobile terminal, comprising the steps of:
a) capturing an image;
b) formatting the image into a specific image format data; and
c) transmitting the specific image format data to an external unit.

15. The method as recited in claim 14, wherein the step b) includes the steps of:
converting the captured image into YUV image data; and
compressing the YUV data by using a specific image codec.

16. The method as recited in claim 15, wherein the specific image format data is one of JPEG image data and MPEG image data.
